# FASCICULE DE BREVET EUROPEEN

(11) **EP 1 984 929 B1**
(45) Date de publication et mention de la délivrance du brevet: **10.04.2013**
(21) Numéro de dépôt: 07730939.1
(22) Date de dépôt: 07.02.2007
(51) Int. Cl.: H01F 1/00, H01F 1/44, A61K 49/18, B82Y 25/00, B82Y 5/00

(54) **PREPARATION DE NANOPARTICULES DE MAGHEMITE ENROBEES DE POLY(OXYDE D'ETHYLENE)**
HERSTELLUNG VON MIT POLY(ETHYLENOXID) ÜBERZOGENEN MAGHEMIT-NANOTEILCHEN
PREPARATION OF POLY(ETHYLENE OXIDE)-COATED NANOPARTICLES OF MAGHEMITE

(30) Priorité: 07.02.2006 FR 0601076
(43) Date de publication de la demande: 29.10.2008
(73) Titulaire: CENTRE NATIONAL DE LA RECHERCHE SCIENTIFIQUE (CNRS), 75016 Paris (FR); Universite De Haute Alsace, 68093 Mulhouse Cedex (FR)
(72) Inventeur: DELAITE, Christelle, F-68720 Froeningen (FR); FLESH, Christophe, F-68350 Brunstatt (FR); DUMAS, Philippe, F-88230 Fraize (FR)
(74) Mandataire: Blot, Philippe Robert Emile
(86) Numéro de dépôt international: PCT/FR2007/000223
(87) Numéro de publication internationale: WO 2007/090962

(56) Documents cités:
- FR-A1- 2 855 315
- US-A- 5 916 539
- US-A1- 2006 018 835
- CH. FLESCH ET AL.: "Poly(ethylene glycol) Surface Coated Magnetic particles" MACROMOL. RAPID COMMUN., vol. 26, 9 juin 2005 (2005-06-09), pages 1494-1498, XP002402412
- SHIGEO AOYAMA ET AL.: "Adsorption of silane coupling agent on Co-gamma-Fe2O3 and its effect on dispersability" JOURNAL OF MATERIALS SCIENCE, vol. 23, 1988, pages 1729-1734, XP008069952
- BUTTERWORTH M D ET AL: "PREPARATION OF ULTRAFINE SIICA- AND PEG-COATED MAGNETITE PARTICLES", COLLOIDS AND SURFACES. A, PHYSICACHEMICAL AND ENGINEERING ASPECTS, ELSEVIER, AMSTERDAM, NL, vol. 179, 1 January 2001 (2001-01-01), pages 93-102, XP008069721, ISSN: 0927-7757, DOI: 10.1016/S0927-7757(00)00633-6

## Description

La présente invention concerne des nanoparticules comprenant un coeur de maghémite et une écorce de poly(oxyde d'éthylène), ainsi qu'un procédé pour leur préparation.

Certains traitements médicaux impliquent l'injection de particules dans le sang d'un patient. Tel est le cas notamment pour des agents de contraste pour l'IRM. Il en sera de même à l'avenir pour les médiateurs pour hyperthermie magnétique. Les particules sont généralement injectées sous forme de dispersions colloïdales, et il est nécessaire que ces dispersions soient stables.

Une dispersion peut être stabilisée soit par répulsion électrostatique, soit par répulsion stérique.

Une dispersion stabilisée par répulsion électrostatique ne peut être utilisée pour une injection, car les particules chargées seraient éliminées immédiatement par le système immunitaire. En outre, l'oxyde de fer présente un point isoélectrique (valeur du pH pour laquelle la densité de charge de surface est nulle) de 7. Ainsi, dans les conditions de pH physiologique, les nanoparticules d'oxyde de fer ne sont pas chargées et donc précipitent.

Une dispersion stabilisée par répulsion stérique est obtenue notamment par greffage de macromolécules hydrophiles à la surface des particules.

On connaît des particules d'oxyde de fer enrobées par une couche de polymère hydrophile, tel que le dextran. Ces particules sont utilisées pour l'IRM du foie ou des ganglions lymphatiques. Elles sont généralement désignées par (U)SPIO [(Ultrasmall)SuperParamagnetic Iron Oxide] et elles sont décrites notamment par Neuberger, et al. [J. Magn. Magn. Mat., 293 (2005) 483]. Elles sont préparées par coprécipitation de l'oxyde de fer directement dans une solution aqueuse du polymère préformé tel que décrit dans US-4,452,773. Dans ces matériaux, il n'existe pas de liaison covalente entre la maghémite et le polymère. L'enrobage de polymère est donc sensible aux phénomènes de désorption /déplétion. Pour améliorer la qualité de ces particules, il a été proposé de réticuler le dextran autour des nanoparticules (US-5,262,176) ou de réticuler le dextran grâce aux nanoparticules d'oxyde de fer préalablement modifiées en surface par un dérivé de type aminosilane (FR-2,855,315). Le principal inconvénient de ces procédés de l'art antérieur est le nombre d'étapes de la modification chimique et le fait que chaque étape de modification nécessite une étape de purification très longue, car essentiellement effectuée par dialyse. En outre, le dextran n'est pas très efficace pour limiter l'adsorption des protéines plasmatiques et donc allonger la demi-vie des nanoparticules.

Il est connu que le poly(oxyde d'éthylène) POE est un agent de surface efficace pour augmenter la demi-vie plasmatique de particules, notamment lorsqu'il est sous forme téléchélique, par exemple un polyéthylène glycol PEG. Cependant, le taux de greffage de POE à la surface des nanoparticules est médiocre. En effet, l'encombrement stérique résultant des chaînes polymères autour d'une particule limite la quantité de polymère qui se fixe sur la particule du fait que l'accès aux sites actifs de la surface de la particule est limité. Par exemple, Butterworth et al., [Surf. A, 179, 93 (2001)], décrivent de telles particules pour lesquelles la teneur maximale en PEG est de 9% massique. En outre, Davis et al., [Coll Surf A : Physicochem. Eng. Aspects, 179 (2001) 93], décrivent le greffage sur des particules d'oxyde magnétique, de chaînes poly(oxyde d'éthylène) (POE) préformées et fonctionnalisées par un groupe organosilane, Cette technique présente les mêmes inconvénients que celle décrite par Butterworth précité.

L'article de Flesch et al. [Macromol. Rapid Commun. 26 (2005) 1494] décrit le couplage sur des particules de maghémite de polyéthylène glycol méthacrylate en présence d'un catalyseur.

En outre, les chaînes préformées sont généralement obtenues par un procédé utilisant un catalyseur, et elles contiennent des résidus de ce catalyseur qui peuvent être toxiques pour des applications médicales des particules modifiées.

Le but de la présente invention est de proposer un procédé plus simple et sans catalyseur pour greffer des macromolécules à la surface de nanoparticules de maghémite en obtenant un taux de greffage important, afin d'obtenir des nanoparticules qui ont un enrobage de polymère fixé par liaison covalente (donc de manière stable) sur un coeur de maghémite et qui sont exemptes de résidus toxiques.

En conséquence, la présente invention a pour objet un procédé de préparation d'un matériau constitué par une dispersion aqueuse de nanoparticules de maghémite enrobées par du poly(éthylène glycol) POE, ainsi que le matériau obtenu.

Le procédé selon la présente invention est défini dans la revendication 1.

Plus précisément, le procédé consiste à :
- préparer une dispersion aqueuse de nanoparticules de maghémite;
- modifier les nanoparticules de maghémite en dispersion par un agent de couplage choisi parmi les alcoxysilanes portant un groupement R ;
- faire polymériser l'oxyde d'éthylène en présence des nanoparticules de maghémite silylées obtenues à l'étape précédente.

Un agent de couplage utilisable dans le procédé de la présente invention est un alcoxysilane, notamment un mono-, di- ou de préférence un trialcoxysilane comprenant un groupement R qui permet d'amorcer la polymérisation de l'oxyde d'éthylène.

Le groupement R est de préférence un groupe hydroxyle ou un précurseur de groupe hydroxyle. Comme exemple de précurseur de groupe hydroxyle, on peut citer le groupe époxyde.

Les groupements alkyle d'un agent de couplage ont de préférence de 1 à 4 atomes de carbone, et préférentiellement de 1 à 2 atomes de carbone. Les groupes alcoxy d'un agent de couplage peuvent être identiques ou différents.

La modification des nanoparticules de maghémite est effectuée dans un solvant aqueux ou dans un solvant organique. Lorsqu'un solvant organique anhydre est utilisé, il est choisi de préférence parmi les solvants aprotiques polaires, par exemple DMF. Lorsque le solvant n'est pas anhydre, il peut être choisi parmi les solvants protiques, par exemple l'éthanol.

Comme exemple d'agent de couplage, on peut citer les composés du type hydroxysilane dans lesquels R est OH, par exemple l'hydroxyméthyltriéthoxysilane, le N-(hydroxyéthyl)-N-méthylaminopropyl-triméthoxysilane et le bis(2-hydroxy-éthyl)-3-aminopropyl-triéthoxysilane. On peut en outre citer les composés du type glycidoxypropylsilane dans lesquels le substituant R est un groupe époxyde, par exemple le 3-(glycidoxypropyl)triméthoxysilane, le (3-glycidoxypropyl)-diméthyléthoxysilane, le (3-glycidoxypropyl)méthyldiéthoxy-silane, et le (3-glycidoxypropyl)méthyldiméthoxysilane.

La dispersion de maghémite peut être préparée par des procédés décrits dans l'art antérieur.

Par exemple un procédé tel que décrit par R. Massart (IEEE Transactions on Magnetics, Vol. MAG-17, N° 2, mars 1981) consiste à préparer une solution aqueuse de FeCl₃ et de FeCl₂ acidifiée par HCl, à ajouter cette solution à une solution aqueuse d'ammoniaque, à séparer sans lavage par centrifugation ou par décantation, le précipité formé. Ensuite, le précipité est :
- soit mis en contact avec une solution aqueuse d'hydroxyde de tétraméthylammonium,
- soit mis en contact avec une solution aqueuse d'acide perchlorique, puis isolé par centrifugation.

Les nanoparticules isolées sont ensuite redispersées dans l'eau, de préférence en quantité telle que la concentration en nanoparticules de maghémite dans l'eau soit comprise entre 5 et 10% massique.

Un autre procédé de préparation de nanoparticules de maghémite est décrit par S. Mornet, et al. [J. Magn. Mat, 293 (2005) 127]. Il consiste 1) à faire précipiter des nanoparticules de magnétite dans une solution aqueuse en ajoutant une solution aqueuse de FeCl₂ acidifiée par HCl à une solution aqueuse de FeCl₃, puis en ajoutant de l'ammoniaque, sous agitation, 2) à éliminer le surnageant après la formation du précipité, 3) à oxyder partiellement le précipité formé en maghémite par addition rapide d'acide nitrique, 4) à oxyder totalement par addition d'une solution de nitrate de fer à chaud, puis à laver le solide obtenu par des cycles centrifugation/redispersion, et 5) à peptiser par addition d'acide nitrique.

Dans un mode de réalisation particulier, l'étape de modification des nanoparticules comprend les phases suivantes :
- mélange sous agitation d'une dispersion aqueuse de maghémite et d'une solution d'agent de couplage, à une température comprise entre 20 et 100°C pendant une durée comprise entre 3 et 20 heures ;
- hydrolyse des groupements alcoxy de l'agent de couplage et du précurseur du groupement réactif R, le cas échéant ;
- extraction des nanoparticules par centrifugation ;
- séchage sous vide dynamique à une température comprise entre 20 et 150°C pendant une durée comprise entre 3 et 24 heures.

Dans un mode de réalisation particulier, l'étape de fixation de chaînes POE sur les nanoparticules modifiées par l'agent de couplage est effectuée sous atmosphère inerte dans un solvant anhydre apolaire, et elle comprend les phases suivantes :
- introduction dans un réacteur, d'un solvant apolaire anhydre et des nanoparticules de maghémite modifiées par l'agent de couplage puis traitées pour les rendre anhydres ;
- addition progressive d'oxyde d'éthylène sous vide partiel et sous agitation, le réacteur étant maintenu à une température de -35°C à -10°C, pendant une durée de 15 à 30 minutes ;
- maintien du mélange réactionnel pendant une durée comprise entre 10 et 100 h à une température comprise entre 40 et 100°C (par exemple 90°C / 50 h)
- extraction des nanoparticules par centrifugation et lavage, puis dispersion dans l'eau.

Le solvant apolaire anhydre est avantageusement le toluène.

Le matériau obtenu par le procédé de l'invention est une dispersion aqueuse d'agrégats de nanoparticules élémentaires, et il est caractérisé en ce que :
- une nanoparticule élémentaire comprend un coeur de maghémite et un enrobage de POE ;
- le coeur de maghémite est constitué par un agrégat de nanoparticules de maghémite ayant un diamètre de 7 à 10 nm, mesuré par microscopie électronique à transmission ;
- le coeur de maghémite a un diamètre de 40 à 50 nm, mesuré par diffusion dynamique de la lumière ;
- les agrégats de nanoparticules élémentaires ont un diamètre de 50 nm à 90 nm, mesuré par diffusion dynamique de la lumière ;
- le rapport en poids POE/maghémite des agrégats de nanoparticules élémentaires est de 80 à 850 mg/g correspondant à un rapport en poids POE/agrégats de 7 à 46% en poids.

Une dispersion formant le matériau selon la présente invention peut être utilisée avantageusement comme agent de contraste notamment pour l'IRM.

La présente invention est expliquée plus en détail par l'exemple suivant, qui est donné à titre d'illustration et auquel l'invention n'est pas limitée.

### Exemple

### Préparation d'une dispersion de maghémite

Dans chacune des étapes du procédé de préparation de la dispersion de maghémite, l'eau utilisée est de l'eau ultra-pure de qualité HPLC, afin d'éviter la présence d'ions floculants.

Au cours d'une première étape, on forme un précipité de magnétite, selon le mode opératoire suivant. On prépare une solution aqueuse de FeCl₃ en introduisant sous agitation (500 rpm) 10,87 g de FeCl₃ (0,039 mol) dans un réacteur de 1 L muni d'une agitation mécanique et d'un reflux, et contenant 435 mL d'eau à 80°C. Lorsque FeCl₃ est totalement dissous, on ajoute une solution de 3,92 g de FeCl₂ (0,0195 mol) préalablement dissous dans 22 mL d'HCl 1,5 M, et on agite jusqu'à ce que la couleur soit uniforme. On augmente alors la vitesse d'agitation à 850 rpm et on ajoute très rapidement 45 mL d'ammoniaque au milieu réactionnel. Il se forme instantanément un précipité noir de magnétite. On laisse le milieu réactionnel sous agitation pendant 15 min, puis on l'extrait du réacteur et on laisse décanter dans un bécher placé sur une plaque magnétique. Ensuite, on élimine le surnageant par aspiration en enlevant le maximum de liquide, en maintenant les nanoparticules de magnétite obtenues à l'abri de l'air.

Au cours d'une deuxième étape, on effectue une oxydation partielle de la magnétite en maghémite, et l'échange des contre-ions NH₄⁺ par NO₃⁻ selon le mode opératoire suivant. A température ambiante, ajouter 70 mL d'acide nitrique 2 M, agiter 2 à 3 min puis laisser décanter 10 min. Eliminer le surnageant. L'oxyde de fer se dissolvant dans ces conditions d'acidité, il faut réaliser cette étape le plus rapidement possible afin de limiter les pertes.

Au cours d'une troisième étape, on effectue une oxydation totale, selon le mode opératoire suivant. On prépare une solution oxydante de nitrate de fer (0,33 M) en dissolvant 10 g de nitrate de fer dans 75 mL d'eau chauffée à 100°C. On verse la solution chaude sur la maghémite et le maintien à 100°C pendant 30 min. Ensuite on arrête le chauffage et on laisse refroidir et décanter. On lave le solide au cours de trois cycles de centrifugation-redispersion (8 400 tours.min⁻¹, 2 min) dans l'acétone afin d'éliminer les ions chlorure et nitrate résiduels.

Au cours d'une quatrième étape, on effectue une peptisation du solide, selon le mode opératoire suivant. On ajoute 50 mL d'acide nitrique 2 M au solide obtenu à la fin de l'étape précédente, on agite pendant 15 min puis on centrifuge. Après la centrifugation, on élimine le surnageant et on effectue à nouveau 3 lavages à l'acétone. Ensuite, on redisperse la maghémite dans 60 mL d'eau et on enlève les traces d'acétone au Rotovapor à 80°C, jusqu'au début de l'élimination d'eau. Ensuite on transvase le ferrofluide obtenu dans un flacon de 60 mL et on complète avec de l'eau. On traite la dispersion pendant 3 minutes par une sonde à ultrasons pour désagréger au maximum les nanoparticules.

Les caractéristiques de la dispersion de maghémite ainsi obtenue sont rassemblées dans le tableau I ci-déssous.

**Tableau I**

| Paramètre | Technique d'analyse | Résultat |
|---|---|---|
| Concentration en maghémite | Dosage volumétrique | 79 g/L |
| Point isoélectrique | Zêtamétrie | 6,5 |
| Domaine de floculation | Visuel | 5<pH<9 |
| Concentration en ions Cl⁻ entraînant la floculation | Spectrophotométrie visible | 0,05 M de NaCl à pH 4,5 |
| pH naturel | pH mètre | 2,5 |
| Taille des nanoparticules | Microscopie électronique à transmission | 11±3 nm |
| | Diffusion dynamique de la lumière | 38 ± 5 nm à pH 5 |
| Surface spécifique | Adsorption d'azote (BET) | 130 m²/g |
| Aimantation | Magnétomètre à SQUID * | 49 emu/g à 5 000 Oe |

| | | |
|---|---|---|
| * : Superconducting Quantum Interference Device (Détecteur supraconducteur à interférence quantique). | | |

### Modification des nanoparticules de maghémite en dispersion

Au cours d'une première étape, on a hydrolysé le groupement époxy du 3-(glycidoxypropyl)triméthoxysilane dans 50 mL d'eau acidifiée à pH 2 à l'aide d'une solution d'acide sulfurique 0,05 M, selon le mode opératoire suivant. Dans un ballon surmonté d'un réfrigérant, on a chauffé sous reflux le silane (2,22 g, 9,2 mmol) et la solution acide à l'aide d'un bain d'huile pendant une heure. Après refroidissement, on a ajusté le pH du milieu à 5 par addition d'une solution d'acétate de sodium 0,5 M. Parallèlement, 20 g de ferrofluide (correspondant à 1,7 g de maghémite) sont également ajustés à pH 5, puis ajoutés à la solution de silane. La dispersion est ensuite portée à reflux pendant 3,5 heures. Après refroidissement, la dispersion est centrifugée (20 min, 8400 tr.min⁻¹) et l'excès de silane éliminé par cinq cycles de centrifugation-dispersion dans l'eau. La maghémite silylée est finalement séchée sous vide dynamique à 70°C pendant 24 heures, cette dernière étape provoquant la formation de liaisons covalentes entre le silane et la maghémite.

La quantité de silane immobilisé à la surface des nanoparticules de maghémite, déterminée par analyse thermo-gravimétrique, est de 0,5 mmol/g de maghémite.

### Fixation de chaînes polymère

On a introduit 0,5 g de maghémite modifiée dans un ballon de 500 mL et on a traité sous vide dynamique à 130°C pendant 3 heures. Ensuite, on a ajouté 160 mL de toluène sec sous courant d'azote et une ampoule contenant l'oxyde d'éthylène (OE) liquéfié est fixée sur le ballon. La dispersion de maghémite dans le toluène est refroidie à l'aide d'un bain froid (constitué par un mélange éthanol/isopro-panol refroidi à l'azote liquide) afin de mettre le dispositif sous vide statique à l'aide d'une pompe à palettes. L'OE (10 mL, 0,2 mol) est alors ajouté progressivement, afin d'éviter les surpressions dans le dispositif, et le milieu réactionnel maintenu dans le bain froid est agité pendant 30 minutes. Ensuite, on chauffe à 50°C à l'aide d'un bain d'huile pendant 95 heures. Après réaction, la dispersion est centrifugée et le solide lavé alternativement 3 fois à l'eau, 3 fois au THF et 3 fois au méthanol. Les tubes de centrifugation sont chauffés par air chaud pour faciliter la redispersion du solide. Une partie du solide est ensuite dispersée dans l'eau et une autre partie est séchée sous vide dynamique à température ambiante pendant 24 heures.

Les caractéristiques des nanocomposites obtenus sont rassemblées dans le tableau II ci-dessous.

**Tableau II**

| Paramètre | Technique d'analyse | Résultat |
|---|---|---|
| Teneur en polymère | Thermogravimétrie | 850 mg/g de maghémite |
| Point isoélectrique | Zêtamétrie | aucun |
| Domaine de floculation | Visuel | aucun |
| Concentration en ions Cl⁻ entraînant la floculation | Spectrophotométrie visible | Stable dans la gamme étudiée jusqu'à 1 M |
| Taille des nanoparticules | Diffusion dynamique de la lumière | 52 ± 6 nm à pH 5 |
| Aimantation | Magnétomètre à SQUID | 26 emu/g à 5 000 Oe |

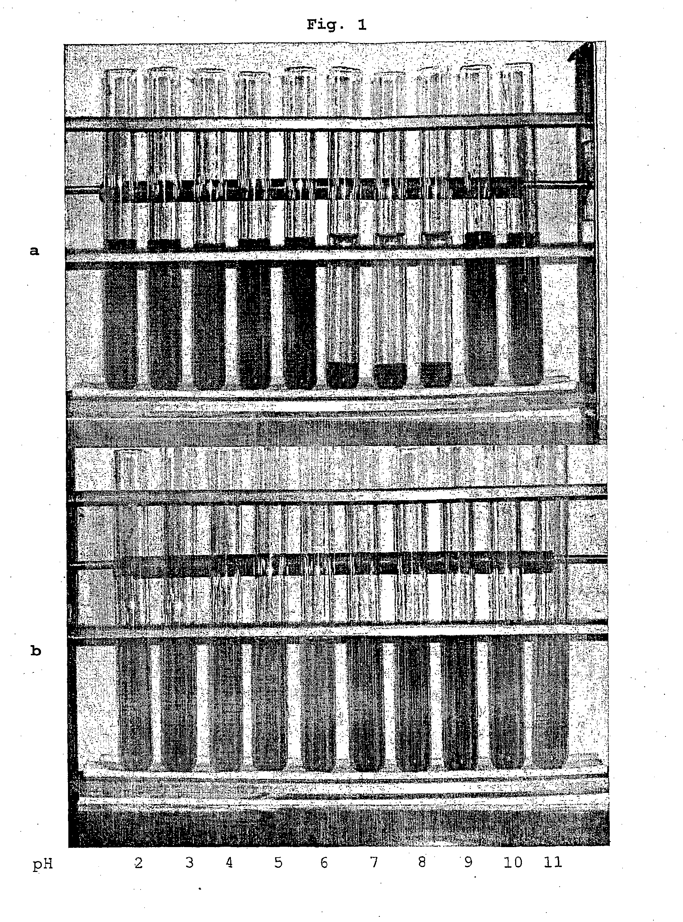

La figure 1 montre le comportement en fonction du pH d'une dispersion de maghémite non modifiée (partie supérieure a), et de la dispersion de maghémite modifiée par greffage de POE préparée dans le présent exemple (partie inférieure b). Il apparaît que les nanoparticules de maghémite non modifiée floculent dans le domaine de pH de 7 à 9, alors que les nanoparticules modifiées restent stables.

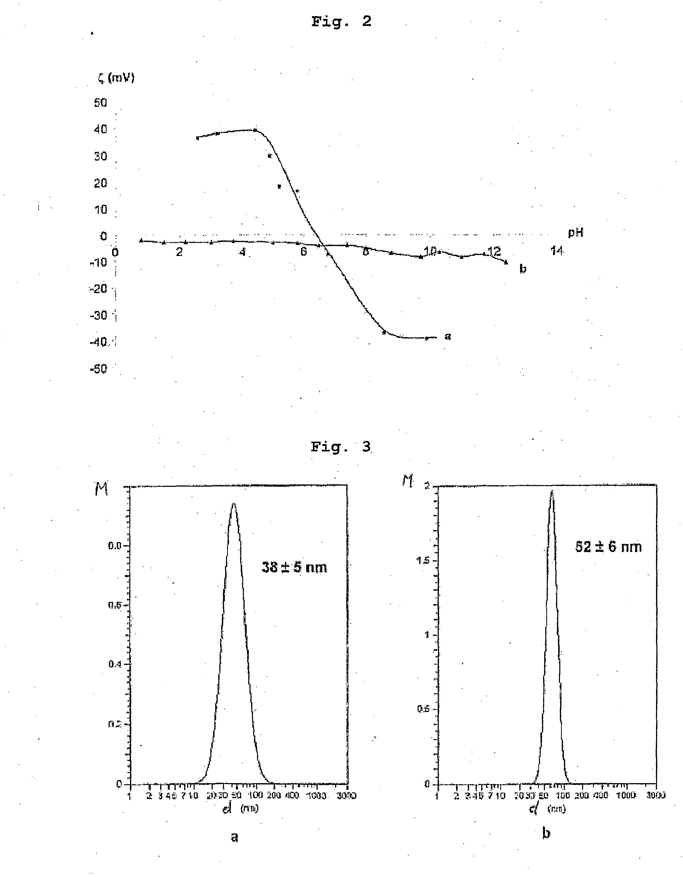

La figure 2 représente l'évolution du potentiel Zêta ξ en fonction du pH de la dispersion de nanoparticules modifiées [courbe a) pour la maghémite non traitée, courbe b) pour la maghémite greffée par le POE]. L'absence d'un point isoélectrique et la faible valeur du potentiel Zêta quel que soit le pH confirment le caractère stérique de la stabilisation. La valeur du potentiel est de -4,5 mV à pH 7,4, qui est le pH sanguin.

La figure 3 représente la distribution moyenne en taille. La proportion M est donnée en ordonnée, la dimension d (en nm) est donnée en abscisse. La courbe a) correspond aux nanoparticules de maghémite non modifiée dispersées dans l'eau distillée, à leur pH naturel, et donc stabilisées par voie électrostatique. La courbe b) correspond à la dispersion de maghémite modifiée obtenue dans le présent exemple.

Il apparaît que les tailles restent faibles après greffage de POE.

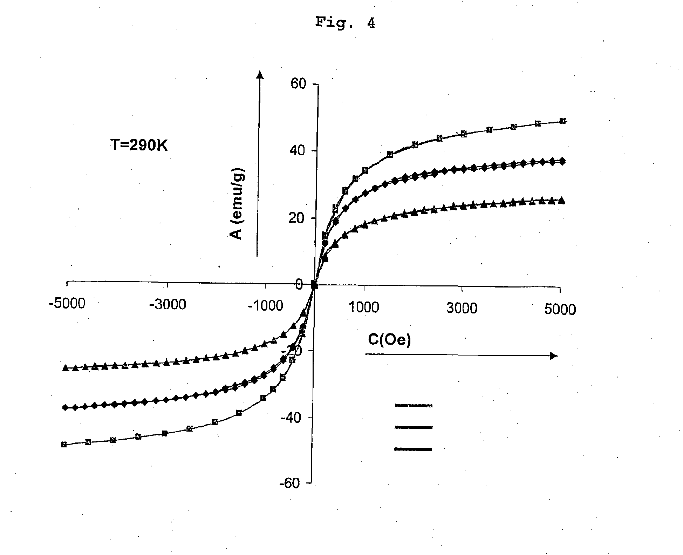

La figure 4 représente l'aimantation en fonction du champ magnétique de la maghémite non modifiée (courbe matérialisée par 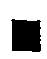 ), de la maghémite modifiée par le silane (courbe matérialisée par ◆) et de la maghémite, modifiée par le POE (courbe matérialisée par ▲). Sur ces courbes, le champ magnétique C (en Oerstedt) est donné en abscisse, et l'aimantation A (emu/g) est donnée en ordonnée. L'absence d'hystérèse confirme que la maghémite modifiée par le POE possède un comportement superparamagnétique à température ambiante.

## Revendications

1. Procédé pour la préparation d'un matériau constitué par une dispersion aqueuse de nanoparticules de maghémite enrobées de poly(oxyde d'éthylène) POE, **caractérisé en ce qu'**il consiste à polymériser l'oxyde d'éthylène en présence de nanoparticules de maghémite modifiées par un agent de couplage comprenant un groupement alcoxysilyle et un groupement R, puis traitées pour les rendre anhydres, ladite polymérisation étant amorcée à partir des groupements réactifs R qui sont capables d'amorcer la polymérisation de l'oxyde d'éthylène sans addition de catalyseur, et qui sont fixés sur les nanoparticules de maghémite en dispersion par l'intermédiaire de l'agent de couplage.

2. Procédé selon la revendication 1, **caractérisé en ce qu'**il consiste à:
• préparer une dispersion aqueuse de nanoparticules de maghémite ;
• modifier les nanoparticules de maghémite en dispersion par un agent de couplage choisi parmi les alcoxysilanes portant un groupement R ;
• faire polymériser l'oxyde d'éthylène en présence des nanoparticules de maghémite modifiées obtenues à l'étape précédente.

3. Procédé selon l'une des revendications 1 ou 2, **caractérisé en ce que** l'agent de couplage est un alcoxysilane comprenant un groupement R qui permet d'amorcer la polymérisation de l'oxyde d'éthylène.

4. Procédé selon la revendication 3, **caractérisé en ce que** le groupement R est un groupe hydroxyle ou un précurseur de groupe hydroxyle.

5. Procédé selon l'une des revendications 3 ou 4, **caractérisé en ce que** les groupements alkyle de l'alcoxysilane ont de 1 à 4 atomes de carbone.

6. Procédé selon l'une des revendications 1 à 5, dans lequel le groupement alcoxysilyle est un groupement trialcoxysilyle.

7. Procédé selon la revendication 2, **caractérisé en ce que** la modification des nanoparticules de maghémite est effectuée dans un solvant aqueux ou dans un solvant organique.

8. Procédé selon la revendication 2, **caractérisé en ce que** l'étape de modification des nanoparticules comprend les phases suivantes :
• mélange sous agitation d'une dispersion aqueuse de maghémite et d'une solution d'agent de couplage, à une température comprise entre 20 et 100°C pendant une durée comprise entre 3 et 20 heures ;
• extraction des nanoparticules par centrifugation ;
• séchage sous vide dynamique à une température comprise entre 20 et 150°C pendant une durée comprise entre 3 et 24 heures.

9. Procédé selon l'une des revendications 1 à 8, **caractérisé en ce que** l'étape de fixation de chaînes POE sur les nanoparticules modifiées par l'agent de couplage est effectuée sous atmosphère inerte dans un solvant anhydre apolaire, et elle comprend les phases suivantes :
• introduction dans un réacteur, d'un solvant apolaire anhydre et des nanoparticules de maghémite modifiées par l'agent de couplage puis traitées pour les rendre anhydres ;
• addition progressive d'oxyde d'éthylène sous vide partiel et sous agitation, le réacteur étant maintenu à une température de -35°C à -10°C, pendant une durée de 15 à 30 minutes ;
• maintien du mélange réactionnel pendant une durée comprise entre 10 et 100 h à une température comprise entre 40 et 100°C ;
• extraction des nanoparticules par centrifugation et lavage, puis dispersion dans l'eau.

10. Procédé selon l'une des revendications 1 à 9, **caractérisé en ce que** la polymérisation de l'oxyde d'éthylène en présence de nanoparticules de maghémite est réalisée dans le toluène.

11. Matériau susceptible d'être obtenu par le procédé selon les revendications 1 à 10, constitué par une dispersion aqueuse d'agrégats de nanoparticules élémentaires, **caractérisé en ce que** :
• une nanoparticule élémentaire comprend un coeur de maghémite et un enrobage de POE ;
• le coeur de maghémite d'une nanoparticule élémentaire est constitué par un agrégat de nanoparticules d'oxyde de fer ayant un diamètre de 7 à 10 nm, mesuré par microscopie électronique à transmission ;
• le coeur de maghémite d'une nanoparticule élémentaire a un diamètre de 40 à 50 nm, mesuré par diffusion dynamique de la lumière ;
• les agrégats de nanoparticules élémentaires ont un diamètre de 50 nm à 90 nm, mesuré par diffusion dynamique de lumière ;
• le rapport en poids POE/maghémite des agrégats de nanoparticules élémentaires est de 80 à 850 mg/g correspondant à un rapport en poids POE/agrégats de 7 à 46% en poids.

12. Agent de contraste pour l'IRM, **caractérisé en ce qu'**il comprend un matériau selon la revendication 11.

## Claims

1. Process for the preparation of a material formed by an aqueous dispersion of maghemite nanoparticles coated with polyethylene oxide (PEO), **characterised in that** it consists of polymerising ethylene oxide in the presence of maghemite nanoparticles modified by a coupling agent comprising an alcoxysilyl group and an R group then treated by rendering them anhydrous, wherein said polymerisation is initiated starting from reactive R groups, which are capable of initiating the polymerisation of ethylene oxide without adding a catalyst and which are fixed to the maghemite nanoparticles in dispersion by means of the coupling agent.

2. Process according to claim 1, **characterised in that** it consists of:
● preparing an aqueous dispersion of maghemite nanoparticles;
● modifying the maghemite nanoparticles in dispersion by a coupling agent selected from alcoxysilanes carrying an R group;
● causing the ethylene oxide to polymerise in the presence of the modified maghemite nanoparticles obtained in the preceding step.

3. Process according to one of claims 1 or 2, **characterised in that** the coupling agent is an alcoxysilane containing an R group, which enables initiation of the polymerisation of ethylene oxide.

4. Process according to claim 3, **characterised in that** the R group is a hydroxyl group or a hydroxyl group precursor.

5. Process according to one of claims 3 or 4, **characterised in that** the alkyl groups of the alcoxysilane have 1 to 4 carbon atoms.

6. Process according to one of claims 1 to 5, in which the alcoxysilyl group is a trialcoxysilyl group.

7. Process according to claim 2, **characterised in that** the modification of the maghemite nanoparticles is conducted in an aqueous solvent or in an organic solvent.

8. Process according to claim 2, **characterised in that** the step of modifying the nanoparticles comprises the following phases:
● mixing an aqueous dispersion of maghemite and a solution of the coupling agent with agitation at a temperature in the range of between 20 and 100°C for a period ranging between 3 and 20 hours;
● extracting the nanoparticles by centrifugation;
● drying under dynamic vacuum at a temperature in the range of between 20 and 150°C for a period ranging between 3 and 24 hours.

9. Process according to one of claims 1 to 8, **characterised in that** the step of fixing PEO chains to the modified nanoparticles by the coupling agent is conducted in an inert atmosphere in an anhydrous non-polar solvent and comprises the following phases:
● inserting into a reactor an anhydrous non-polar solvent and the maghemite nanoparticles modified by the coupling agent and then treated to render them anhydrous;
● progressively adding ethylene oxide under partial vacuum and with agitation, wherein the reactor is kept at a temperature of -35°C to -10°C for a period of 15 to 30 minutes;
● keeping the reaction mixture at a temperature in the range of between 40 and 100°C for a period ranging between 10 and 100 h;
● extracting the nanoparticles by centrifuging and washing, then dispersing in water.

10. Process according to one of claims 1 to 9, **characterised in that** the polymerisation of ethylene oxide in the presence of maghemite nanoparticles is conducted in toluene.

11. Material obtainable by the process according to claims 1 to 10, formed by an aqueous dispersion of aggregates of elemental nanoparticles, **characterised in that**:
● an elemental nanoparticle comprises a maghemite core and a PEO coating;
● the maghemite core of an elemental nanoparticle is formed by an aggregate of iron oxide nanoparticles with a diameter of 7 to 10 nm measured by transmission electron microscopy;
● the maghemite core of an elemental nanoparticle has a diameter of 40 to 50 nm measured by dynamic light scattering;
● the aggregates of elemental nanoparticles have a diameter of 50 nm to 90 nm measured by dynamic light scattering;
● the weight ratio of PEO/maghemite of the aggregates of elemental nanoparticles is 80 to 850 mg/g corresponding to a weight ratio of PEO/aggregates of 7 to 46% by weight.

12. MRI contrast agent, **characterised in that** it comprises a material according to claim 11.

## Patentansprüche

1. Verfahren zur Herstellung eines Materials, das aus einer wässrigen Dispersion von Maghemit-Nanopartikeln, die mit Poly(ethylenoxid) PEO umhüllt sind, besteht, **dadurch gekennzeichnet, dass** es darin besteht, Ethylenoxid in Gegenwart von Maghemit-Nanopartikeln, die durch ein Kupplungsmittel, das eine Alkoxysilyl-Gruppierung und eine R-Gruppierung umfasst, modifiziert wurden, dann behandelt wurden, um sie wasserfrei zu machen, zu polymerisieren, wobei die Polymerisierung von den reaktiven R-Gruppierungen aus begonnen wird, die fähig sind, die Polymerisation des Ethylenoxids ohne Zusatz von Katalysator zu starten und die an den Maghemit-Nanopartikeln in Dispersion mit Hilfe des Kupplungsmittels befestigt sind.

2. Verfahren gemäß Anspruch 1, **dadurch gekennzeichnet, dass** es aus:
**●** Herstellen einer wässrigen Dispersion von Maghemit-Nanopartikeln;
● Modifizieren der Maghemit-Nanopartikel in Dispersion durch ein Kupplungsmittel, das aus Alkoxysilanen, die eine R-Gruppierung tragen, ausgewählt ist;
● Polymerisieren-Lassen des Ethylenoxids in Gegenwart der modifizierten Maghemit-Nanopartikel, die in der vorhergehenden Stufe erhalten wurden,
besteht.

3. Verfahren gemäß einem der Ansprüche 1 oder 2, **dadurch gekennzeichnet, dass** das Kupplungsmittel ein Alkoxysilan ist, das eine R-Gruppierung umfasst, die es erlaubt, die Polymerisation des Ethylenoxids zu starten.

4. Verfahren gemäß Anspruch 3, **dadurch gekennzeichnet, dass** die R-Gruppierung eine Hydroxylgruppe oder eine Hydroxylvorläufergruppe ist.

5. Verfahren gemäß einem der Ansprüche 3 oder 4, **dadurch gekennzeichnet, dass** die Alkyl-Gruppierungen des Alkoxysilans 1 bis 4 Kohlenstoffatome haben.

6. Verfahren gemäß einem der Ansprüche 1 bis 5, in dem die Alkoxysilyl-Gruppierung eine Trialkoxysilyl-Gruppierung ist.

7. Verfahren gemäß Anspruch 2, **dadurch gekennzeichnet, dass** die Modifikation der Maghemit-Nanopartikel in einem wässrigen Lösungsmittel oder in einem organischen Lösungsmittel durchgeführt wird.

8. Verfahren gemäß Anspruch 2, **dadurch gekennzeichnet, dass** die Stufe der Modifikation der Nanopartikel die folgenden Phasen umfasst:
Mischen einer wässrigen Maghemit-Dispersion und einer Kupplungsmittellösung unter Rühren bei einer Temperatur zwischen 20 und 100 °C während einer Dauer, die zwischen 3 und 20 Stunden liegt;
● Extraktion der Nanopartikel durch Zentrifugation;
● Trocknung unter dynamischem Vakuum bei einer Temperatur zwischen 20 und 150 °C während einer Dauer, die zwischen 3 und 24 Stunden liegt.

9. Verfahren gemäß einem der Ansprüche 1 bis 8, **dadurch gekennzeichnet, dass** die Stufe der Befestigung der PEO-Ketten an den modifizierten Nanopartikeln durch das Kupplungsmittel unter inerter Atmosphäre in einem apolaren wasserfreien Lösungsmittel durchgeführt wird und dass sie die folgenden Phasen umfasst:
● Einführung eines apolaren wasserfreien Lösungsmittels und der Maghemit-Nanopartikel, die durch das Kupplungsmittel modifiziert wurden, dann behandelt wurden, um sie wasserfrei zu machen, in einen Reaktor;
● schrittweise Zugabe von Ethylenoxid unter partiellem Vakuum und unter Rühren, wobei der Reaktor bei einer Temperatur von -35 °C bis -10 °C gehalten wird, über einen Zeitraum von 15 bis 30 Minuten;
● Halten des Reaktionsgemischs bei einer Temperatur von zwischen 40 und 100 °C für eine Zeit, die zwischen 10 und 100 h liegt;
● Extraktion der Nanopartikel durch Zentrifugation und Waschen, danach Dispersion in Wasser.

10. Verfahren gemäß einem der Ansprüche 1 bis 9, **dadurch gekennzeichnet, dass** die Polymerisation des Ethylenoxids in Gegenwart von Maghemit-Nanopartikeln in Toluol durchgeführt wird.

11. Material, das durch das Verfahren gemäß den Ansprüchen 1 bis 10 erhalten werden kann, das aus einer wässrigen Dispersion von Aggregaten von elementaren Nanopartikeln besteht, **dadurch gekennzeichnet, dass**,
● ein elementares Nanopartikel einen Maghemit-Kern und eine PEO-Umhüllung umfasst;
● der Maghemit-Kern eines elementaren Nanopartikels aus einem Aggregat von Eisenoxid-Nanopartikeln mit einem Durchmesser von 7 bis 10 nm, gemessen mittels Durchstrahlungs-Elektronenmikroskopie, besteht;
● der Maghemit-Kern eines elementaren Nanopartikels einen Durchmesser von 40 bis 50 nm, gemessen durch dynamische Diffusion des Lichts, hat;
● die Aggregate aus elementaren Nanopartikeln einen Durchmesser von 50 nm bis 90 nm, gemessen durch dynamische Diffusion des Lichts, haben;
● das Gewichtsverhältnis PEO/Maghemit der Aggregate der elementaren Nanopartikel 80 bis 850 mg/g ist, was einem Gewichtsverhältnis PEO/Aggregate von 7 bis 46 Gew.-% entspricht.

12. Kontrastmittel für Magnet-Resonanz-Darstellung (MR), **dadurch gekennzeichnet, dass** es ein Material gemäß Anspruch 11 umfasst.
